# EUROPEAN PATENT SPECIFICATION

(11) **EP 2 725 978 B1**
(45) Date of publication and mention of the grant of the patent: **11.01.2017**
(21) Application number: 12733364.9
(22) Date of filing: 26.06.2012
(51) Int. Cl.: G01N 33/487, A61B 5/145

(54) **HAND-HELD TEST METER WITH ELECTRO-MAGNETIC INTERFERENCE DETECTION CIRCUIT**
TRAGBARES MESSGERÄT MIT DETEKTIONSSCHALTUNG FÜR ELEKTROMAGNETISCHE INTERFERENZEN
APPAREIL DE MESURE DE TEST PORTATIF AVEC CIRCUIT DE DÉTECTION D'INTERFÉRENCES ÉLECTROMAGNÉTIQUES

(30) Priority: 28.06.2011 US 201113170947
(43) Date of publication of application: 07.05.2014
(73) Proprietor: Lifescan, Inc., Wayne, PA 19087 (US)
(72) Inventor: KRAFT, Ulrich, 65719 Hofheim (DE)
(74) Representative: Carpmaels & Ransford LLP
(86) International application number: PCT/US2012/044180
(87) International publication number: WO 2013/003336

(56) References cited:
- EP-A2- 1 143 245
- US-A1- 2007 087 397
- US-A1- 2009 120 810
- US-A1- 2011 121 865

## Description

### BACKGROUND OF THE INVENTION

### Field of the Invention

The present invention relates, in general, to medical devices and, in particular, to test meters and related methods.

### Description of Related Art

The determination (e.g., detection and/or concentration measurement) of an analyte in a fluid sample is of particular interest in the medical field. For example, it can be desirable to determine glucose, ketone bodies, cholesterol, lipoproteins, triglycerides, acetaminophen and/or HbA1c concentrations in a sample of a bodily fluid such as urine, blood, plasma or interstitial fluid. Such determinations can be achieved using a hand-held test meter in combination with analytical test strips (e.g., electrochemical-based analytical test strips).

US 2007/087397 A1 discloses a system and method for processing a test current for an analyte measurement in a fluid using a test strip and a test meter. However, it does not disclose an electromagnetic interference circuit.

US 2011/121865 A1 discloses an apparatus, system and method for protecting a processing system from electromagnetic interference where the interference module is configured to detect when a power level associated with the interference signal is greater than a threshold value.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified top view of a hand-held test meter according to an embodiment of the present invention;
FIG. 2 is a simplified block diagram of various electrical circuit blocks of the hand-held test meter of FIG. 1;
FIG. 3 is a simplified graphical depiction of an electrochemical response measured by a conventional hand-held test meter in the absence of significant electromagnetic interference;
FIG. 4 is a simplified graphical depiction of an electrochemical response measured by a conventional hand-held test meter in the presence of significant electromagnetic interference;
FIG. 5 is simplified electrical schematic and block diagram of an electromagnetic interference detection circuit in operative communication with a microcontroller block as can be employed in hand-held test meters according to embodiments of the present invention;
FIG. 6 is simplified electrical schematic and block diagram of another electromagnetic interference detection circuit in operative communication with a microcontroller block as can be employed in hand-held test meters according to embodiments of the present invention; and
FIG. 7 is a flow diagram depicting stages in a method for employing a hand-held test meter according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein.

In general, hand-held test meters for use with analytical test strips in the determination of an analyte (such as glucose) in a bodily fluid sample (for example, a whole blood sample) according to embodiments of the present invention include a housing, a test meter control circuit block (e.g. a microcontroller block), and an electromagnetic interference detection circuit block with an antenna configured to sense electromagnetic fields (for example, Radio Frequency [RF] fields including pulse modulated RF fields commonly created by GSM cellular phones and DECT cordless phones) of a predetermined frequency or frequency range (for example, electromagnetic fields in the frequency range of 800 MHz to 2200 MHz). In addition, the electromagnetic interference detection circuit block is configured to generate a signal (such as a demodulated signal) representative of an electromagnetic field sensed by the antenna and to provide that signal to the test meter control circuit block. Moreover, the test meter control circuit block is configured to interrupt operation of the hand-held test meter when the signal received from the electromagnetic interference detection circuit block represents a predetermined electromagnetic field that interferes with the hand-held test meter's operation.

Hand-held test meters according to embodiments of the present invention are beneficial in that the test meter's operation can be interrupted (for example, halted and/or modified by the display of a warning message to the hand-held test meter's user) when a predetermined electromagnetic field that deleteriously interferes with the hand-held test meter's operations is detected. For example, electrical circuit blocks within the hand-held test meter (such as, for example, analog-to-digital convertor circuit blocks) may generate a deleteriously noisy signal due to the presence of an external electromagnetic field. The accuracy of analyte determinations based on such a noisy signal can be degraded in comparison to analyte determinations based on a signal without such electromagnetic field generated noise. However, hand-held test meters according to embodiments of the present invention avoid the potential for such accuracy degradation by detecting electromagnetic fields that can interfere with the hand-held test meter's operation and then appropriately interrupting that operation.

A potential source of an electromagnetic field that could conceivably interfere with the accurate operation of a hand-held test meter is the electromagnetic field created by a nearby active cell phone. For example, the electromagnetic field strength directly beside the antenna surface of an active cell phone can be greater than 100 V/m. An electromagnetic field of such strength could interfere with the accurate operation of hand-held test meters including, for example hand-held test meters for the determination of glucose in a whole blood sample. However, hand-held test meters according to embodiments of the present invention include circuit blocks configured to sense electromagnetic fields and to interrupt operation of the hand-held test meter when an electromagnetic field that interferes with the hand-held test meter's operation is detected.

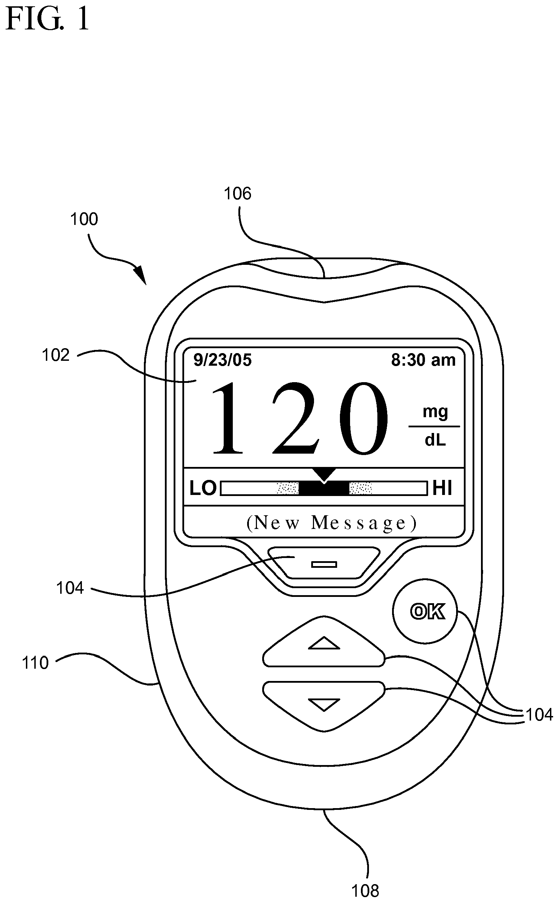

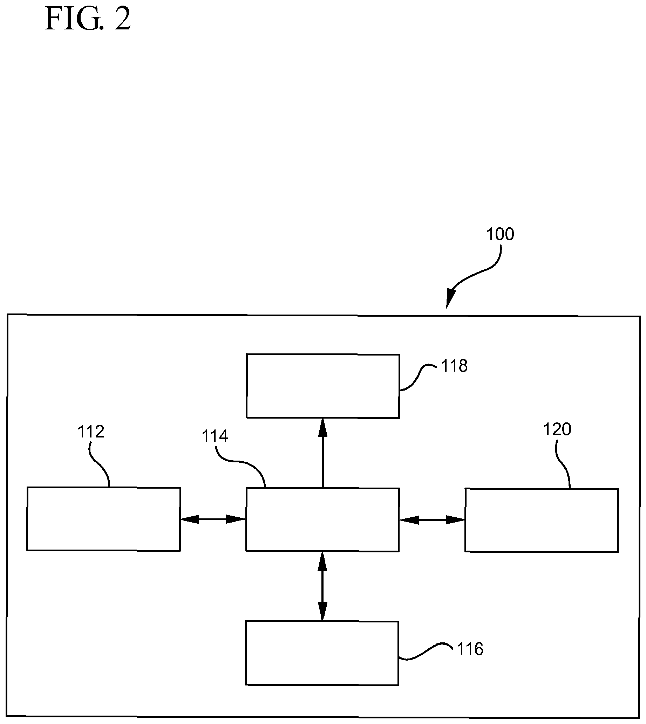

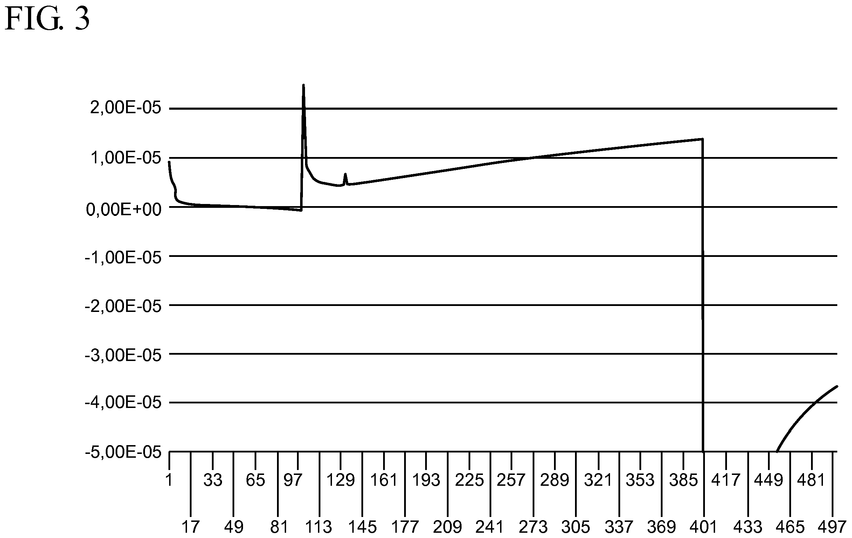

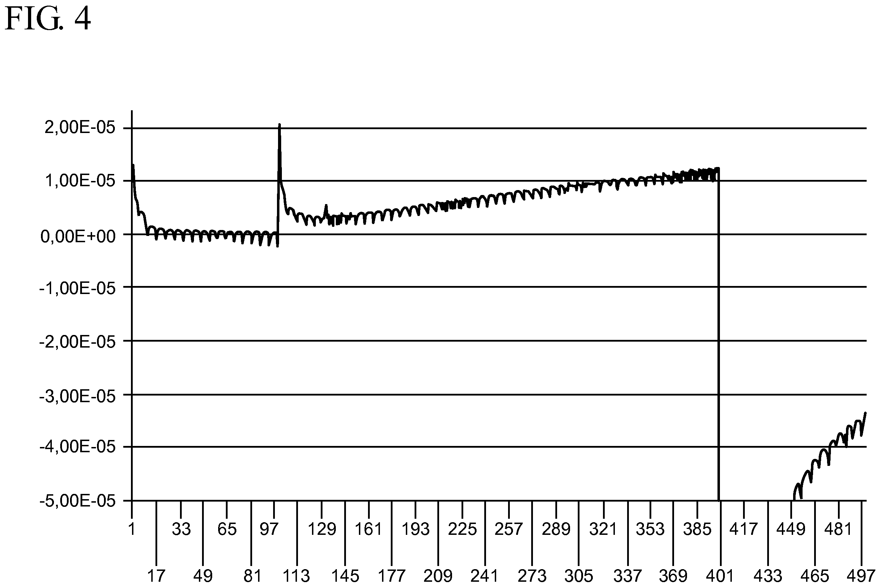

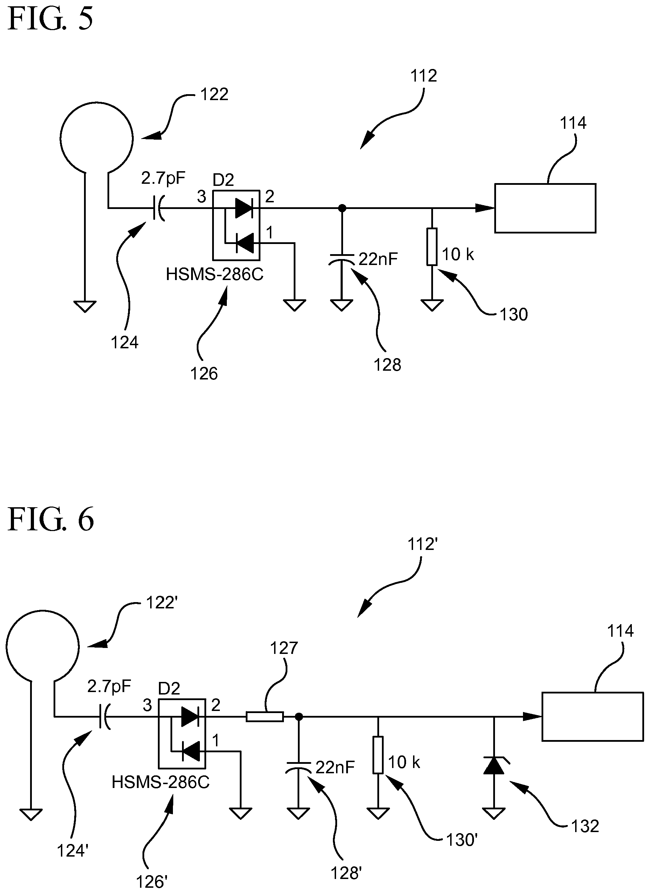

FIG. 1 is a simplified top view depiction of a hand-held test meter 100 with an electromagnetic interference detection circuit (also referred to as an electromagnetic interference detection circuit block) according to an embodiment of the present invention. FIG. 2 is a simplified block diagram of various blocks of the hand-held test meter 100. FIG. 3 is a simplified graphical depiction of an electrochemical response measured by a conventional hand-held test meter in the absence of significant electromagnetic interference. FIG. 4 is a simplified depiction of an electrochemical response measured by a conventional hand-held test meter in the presence of significant electromagnetic interference. FIG. 5 is simplified electrical schematic and block diagram of an electromagnetic interference detection circuit of hand-held test meter 100 in operative communication with a microcontroller block, also of hand-held test meter 100.

Once one skilled in the art is apprised of the present disclosure, he or she will recognize that an example of a hand-held test meter that can be readily modified as a hand-hand test meter according to the present invention is the commercially available OneTouch^{®} Ultra^{®} 2 glucose meter from LifeScan, Inc. (Milpitas, California). Additional examples of hand-held test meters that can also be modified are found in U.S. Patent Application Publications No's. 2007/0084734 (published on April 19, 2007) and 2007/0087397 (published on April 19, 2007) and in International Publication Number WO2010/049669 (published on May 6, 2010).

Hand-held test meter 100 includes a display 102, a plurality of user interface buttons 104, a strip port connector 106, a USB interface 108, and a housing 110 (see FIG. 1). Referring to FIGs. 2 and 5 in particular, hand-held test meter 100 also includes an electromagnetic interference detection circuit block 112, a test meter control circuit block 114 (in the form of a microcontroller block), a communications port block 116, a display control block 118, a memory block 120 and other electronic components (not shown) for applying a test voltage to an analytical test strip (not shown), and also for measuring an electrochemical response (e.g., a plurality of test current values) and determining an analyte based on the electrochemical response. To simplify the current descriptions, the figures do not depict all such electronic circuitry.

Display 102 can be, for example, a liquid crystal display or a bi-stable display configured to show a screen image. An example of a screen image may include a glucose concentration, a date and time, an error message, an electromagnetic interference detection warning message, and a user interface for instructing an end user on how to perform a test.

Strip port connector 106 is configured to operatively interface with the analytical test strip (not depicted in the figures) such as an electrochemical-based analytical test strip configured for the determination of glucose in a whole blood sample. Therefore, the analytical test strip is configured for operative insertion into strip port connector 106. The analytical test strip can be any suitable analytical test strip including an electrochemical-based analytical test strip such as the commercially available OneTouch^{®} Ultra^{®} glucose test strip from LifeScan, Inc. (Milpitas, California). Examples of analytical test strips can be found in U.S. Patent No's. 5,708,247; 5,951,836; 6,241,862; 6,284,125; 6,413,410; 6,733,655; 7,112,265; 7,241,265; and 7,250,105.

USB Interface 108 can be any suitable interface known to one skilled in the art. USB Interface 108 is essentially a passive component that is configured to power and provide a data line to communications port block 116 of hand-held test meter 100.

Once an analytical test strip is interfaced with hand-held test meter 100, or prior thereto, a bodily fluid sample (e.g., a whole blood sample) is dosed into a sample-receiving chamber of the analytical test strip. The analytical test strip can include enzymatic reagents that selectively and quantitatively transforms an analyte into another predetermined chemical form. For example, the analytical test strip can include an enzymatic reagent with ferricyanide and glucose oxidase so that glucose can be physically transformed into an oxidized form.

Memory block 120 of hand-held test meter 100 includes a suitable algorithm that determines an analyte based on the electrochemical response of the analytical test strip.

FIG. 3 is a simplified graphical depiction of an electrochemical response measured by a conventional hand-held test meter in the absence of significant electromagnetic interference with the x-axis having the units of time in 10ms increments and the y-axis having the units of micro-amps. It should be noted that the electrical response of FIG. 3 does not exhibit significant electrical noise. In contrast, FIG. 4 is a simplified graphical depiction of an electrochemical response measured by a conventional hand-held test meter in the presence of an electromagnetic field (emanating from an active GSM cell phone directly beside the hand-held test meter) that is interfering with the hand-held test meter's measurement of the electrochemical response with the x-axis again having the units of time in 10ms increments and the y-axis again having the units of micro-amps. It should be noted that the electrochemical response of FIG. 4 exhibits significant electrical noise as a result of the electromagnetic field. This noise is evident from a comparison of the FIG. 3 and FIG. 4 responses.

Electromagnetic interference detection circuit block 112 includes an antenna (see antenna 122 of FIG. 5 and antenna 122' of FIG. 6 described below) configured to sense electromagnetic fields of a predetermined frequency (for example, Radio Frequency (RF) electromagnetic fields with frequencies in the range of 800MHz to 2200 MHz). Moreover, electromagnetic interference detection circuit block 112 is configured to generate a signal representative of an amplitude modulated electromagnetic field sensed by the antenna and to provide the signal to test meter control circuit block 114.

Test meter control circuit block 114 is configured to interrupt operations of the hand-held test meter when the signal received from the electromagnetic interference detection circuit block is representative of a predetermined electromagnetic field that interferes with hand-held test meter operation. Such a predetermined electromagnetic field can be, for example, an electromagnetic amplitude -modulated (AM) or pulse- modulated electromagnetic field with a field strength of greater than 10 V/m and a frequency in the range of 800MHz to 2200 MHz. Test meter control circuit block 114 can be any suitable test meter control circuit block known to one of skill in the art including, for example, a microcontroller block.

Referring to FIG. 5, electromagnetic interference detection circuit block 112 is described in more detail. Electromagnetic interference detection circuit block 112 includes an antenna 122, a first capacitor 124, a diode 126, a second capacitor 128 and a 10,000 ohm resistor 130. Electromagnetic interference detection circuit block 112 is configured to be particularly beneficial in detecting amplitude modulated or pulse modulated electromagnetic fields such as those generated by GSM cell phones or DECT modulated cordless phones.

Antenna 122 can be any suitable antenna known to one skilled in the art including, for example, a 10mm diameter and approximately 12nH loop antenna etched into a printed circuit board (not shown in the FIGs.) of the hand-held test meter. The combination of 2.7pF first capacitor 124 and antenna 122 is configured to sense electromagnetic frequency bands from a GSM / UMTS cell phone in the range of 800MHz to 2200MHz. Diode 126 and second capacitor 128 are configured to serve as an AM demodulator that generates a 217 Hz pulse signal with a 1/8^{th} duty cycle when electromagnetic interference detection circuit block 112 is in the presence of a GSM cell phone created electromagnetic signal. The amplitude of the 217 Hz signal (which is communicated to either of an analog input or a digital input (not shown in the figures) of microcontroller block 114) will be dependent on the electromagnetic field strength.

In the embodiment of FIG. 5, diode 126 is a diode commercially available as part HSMS-286 from Avago Technologies, San Jose, California, USA and 10,000 ohm resistor 130 is configured as a discharge resistor for capacitor 128.

Electromagnetic interference detection circuit block 112 essentially demodulates amplitude modulated fields received by antenna 122 and creates a demodulated output voltage (i.e., a demodulated signal) that is proportional to the RF field strength multiplied by the amplitude modulation level. For example, for a GSM cell phone the amplitude modulation level is 100% (i.e., the RF is either fully on or fully off) with a 217 Hz interval.

FIG. 6 is simplified electrical schematic and block diagram of another electromagnetic interference detection circuit block 112' in operative communication with a microcontroller block 114 as can be employed in hand-held test meters according to embodiments of the present invention. In FIG. 6, like elements with respect to FIG. 5 are designated with a prime (') in the element number.

Electromagnetic interference detection circuit block 112 includes an antenna 122', a first capacitor 124', a diode 126', a second resistor 127, second capacitor 128', a first resistor 130' and a Zener diode 132. Electromagnetic interference detection circuit block 112' is configured to detect AM modulated electromagnetic fields such as those generated by a GSM cell phone. In the embodiment of FIG. 6, diode 126' is a diode commercially available as part HSMS-286 from Avago Technologies, San Jose, California, USA.

Electromagnetic interference detection circuit block 112' functions in a similar manner as that of electromagnetic interference detection circuit block 112. However, the value of second resistor 127 can be selected to match the sensitivity of the electromagnetic interference detection circuit block to a particular type and strength (e.g., field strengths greater than 10V/m) of electromagnetic field that is known to interfere with a hand-held test meter's operation. Resistor 127 forms a voltage divider with resistor 130'. As the higher the resistance value of resistor 127 is increased, the sensitivity of electromagnetic interference detection circuit block 112' to an electromagnetic fields decreases. A typical but non-limiting resistance value for resistor 127 is 200,000 ohms.

Zener diode 132 is configured to protect microcontroller block 114 from being overloaded by a high voltage signal from electromagnetic interference detection circuit block 112' and can be any suitable Zener diode including, for example, a 2.7V Zener Diode for a microcontroller block operated at supply voltage of 3.0V.

In the embodiments of FIGs. 5 and 6, the electromagnetic interference detection circuit block and microcontroller block can, for example, be configured such that an electromagnetic field of greater than approximately 10 V/m will result in an interruption of the hand-held test meter's operation.

Once apprised of the present disclosure, one skilled in the art will recognize that the electromagnetic interference detection circuit blocks depicted in FIGs. 5 and 6 are for descriptive purposes only and that electromagnetic interference detection circuit blocks employed in embodiments of the present invention can take a form that differs in detail from that of FIGs. 5 and 6.

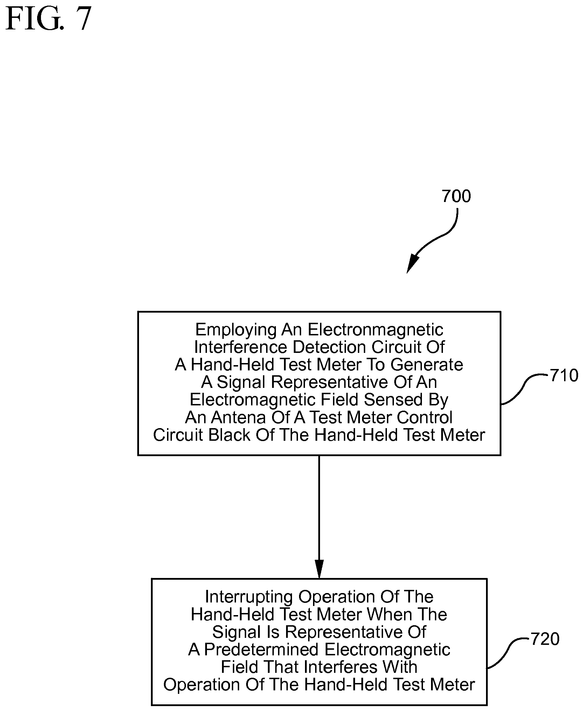

FIG. 7 is a flow diagram depicting stages in a method 700 for employing a hand-held test meter configured for the determination of an analyte (such as glucose) in a bodily fluid sample (for example, a whole blood sample). Method 700 includes employing an electromagnetic interference detection circuit of a hand-held test meter that includes an antenna configured to sense electromagnetic fields of a predetermined frequency to (i) generate a signal representative of an electromagnetic field sensed by the antenna and to (ii) provide the signal to a test meter control circuit block of the hand-held test meter. See step 710 of FIG. 7.

Method 700 also includes interrupting operation of the hand-held test meter when the signal received by the test meter circuit control block from the electromagnetic interference detection block is representative of a predetermined electromagnetic field that interferes with hand-held test meter operation (see step 720 of FIG. 7). For example, the hand-held test meter's operation can be interrupted when the electromagnetic field is of a predetermined frequency (such as a frequency in the range of 800MHz to 2200MHz and/or an electromagnetic field strength of greater than, for example, approximately 10 V/m.

Such interruption can, for example, include displaying an electromagnetic interference warning message to a user via a display of the hand-held test meter. In such a scenario, the hand-held test meter's electromagnetic interference detection circuit and test meter control circuit block, as well as a display control block, are configured to control the display of such a warning message.

Methods according to embodiments of the present invention can, if desired, also include the steps of (i) applying a bodily fluid sample to an electrochemical-based analytical test strip; (ii) measuring an electrochemical response of the electrochemical-based analytical test strip using the hand-held test meter; and (iii) determining the analyte based on the measured electrochemical response. Moreover, once apprised of the present disclosure, one skilled in the art will recognize that method 700 can be readily modified to incorporate any of the techniques, benefits and characteristics of hand-held test meters according to embodiments of the present invention and described herein.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A hand-held test meter (100) for use with an analytical test strip in the determination of an analyte in a bodily fluid sample, the hand-held test meter comprising:
a housing (110);
a test meter control circuit block (114); and
an electromagnetic interference detection circuit block (112) that includes:
an antenna (122) configured to sense electromagnetic fields of a predetermined frequency;
wherein the electromagnetic interference detection circuit block is configured to generate a signal representative of an electromagnetic field sensed by the antenna and to provide the signal to the test meter control circuit block; and wherein the test meter control circuit block is configured to interrupt operations of the hand-held test meter when the signal received from the electromagnetic interference detection circuit block is representative of a predetermined electromagnetic field that interferes with hand-held test meter operation;
wherein said predetermined electromagnetic field is an electromagnetic field which has a field strength greater than 10V/m and either:
has a frequency in the range of 800MHz to 2200MHz; or
is an amplitude modulated Radio Frequency (RF) electromagnetic field and the signal received from the electromagnetic interference detection circuit block is a demodulated signal; or
is a pulse modulated RF electromagnetic field and the signal received from the electromagnetic interference detection circuit block is a demodulated signal.

2. The hand-held test meter of claim 1 wherein the hand-held test meter is configured for the determination of glucose in a whole blood sample using an electrochemical-based analytical test strip.

3. A method for employing a hand-held test meter configured for the determination of an analyte in a bodily fluid sample, the method comprising:
employing an electromagnetic interference detection circuit block, that includes an antenna configured to sense electromagnetic fields of a predetermined frequency, of the hand-held test meter to generate a signal representative of an electromagnetic field sensed by the antenna and to provide the signal to a test meter control circuit block of the hand-held test meter; and
interrupting operation of the hand-held test meter when the signal received by the test meter circuit control block from the electromagnetic interference detection circuit block is representative of a predetermined electromagnetic field that interferes with hand-held test meter operation,
wherein said predetermined electromagnetic field is an electromagnetic field which has a field strength greater than 10V/m and either:
has a frequency in the range of 800MHz to 2200MHz; or
is an amplitude modulated Radio Frequency (RF) electromagnetic field and the signal received from the electromagnetic interference detection circuit block is a demodulated signal; or
is a pulse modulated RF electromagnetic field and the signal received from the electromagnetic interference detection circuit block is a demodulated signal.

4. The method of claim 3 further including:
applying a bodily fluid sample to an electrochemical-based analytical test strip;
measuring an electrochemical response of the electrochemical-based analytical test strip using the hand-held test meter; and
determining the analyte based on the measured electrochemical response.

5. The method of claim 4 wherein the bodily fluid sample is a whole blood sample and the analyte is glucose.

6. The hand-held test meter of claim 1 or the method of claim 3 wherein the test meter control circuit block is a microcontroller block.

7. The method of claim 3 wherein the interrupting step includes interrupting the operation of the hand-held test meter by displaying an electromagnetic interference warning message on a display of the hand-held test meter.

8. The hand-held test meter of claim 1 or the method of claim 3 wherein the electromagnetic interference detection circuit block is further configured as an AM demodulator that produces a 217 Hz signal representative of an electromagnetic field created by a GSM cell phone and sensed by the antenna.

9. The hand-held test meter of claim 6 further including a display and a display module block, and
wherein the microcontroller block, display module block and display are configured to display a warning message to a user when the signal received from the electromagnetic interference detection circuit block is representative of a predetermined electromagnetic field that interferes with hand-held test meter operation.

## Patentansprüche

1. Handgeführtes Prüfmessgerät (100) für die Verwendung mit einem analytischen Teststreifen bei der Bestimmung eines Analyten in einer Körperfluidprobe, wobei das handgeführte Prüfmessgerät umfasst:
ein Gehäuse (110) :
einen Prüfmessgerät-Steuerschaltungsblock (114); und
einen elektromagnetische-Störung-Erkennungsschaltungsblock (112), der umfasst:
eine Antenne (112), gestaltet zum Erfassen elektromagnetischer Felder mit einer vorbestimmten Frequenz;
wobei der elektromagnetische-Störung-Erkennungsschaltungsblock dafür gestaltet ist, ein Signal zu erzeugen, das für ein von der Antenne erfasstes elektromagnetisches Feld repräsentativ ist, und das Signal an den Prüfmessgerät-Steuerschaltungsblock zu übermitteln; und
wobei der Prüfmessgerät-Steuerschaltungsblock dafür gestaltet ist, den Betrieb des handgeführten Prüfmessgeräts zu unterbrechen, wenn das von dem elektromagnetische-Störung-Erkennungsschaltungsblock empfangene Signal für ein vorbestimmtes elektromagnetisches Feld repräsentativ ist, das den Betrieb des handgeführten Prüfmessgeräts stört;
wobei das vorbestimmte elektromagnetische Feld ein elektromagnetisches Feld ist, das eine Feldstärke von höher als 10 V/m aufweist, und entweder:
eine Frequenz in dem Bereich von 800 MHz bis 2200 MHz aufweist; oder
ein amplitudenmoduliertes elektromagnetisches Feld mit Radiofrequenz (RF) ist, und das von dem elektromagnetische-Störung-Erkennungsschaltungsblock empfangene Signal ein demoduliertes Signal ist; oder
ein pulsmoduliertes elektromagnetisches RF-Feld ist, und das von dem elektromagnetische-Störung-Erkennungsschaltungsblock empfangene Signal ein demoduliertes Signal ist.

2. Handgeführtes Prüfmessgerät gemäß Anspruch 1, wobei das handgeführte Prüfmessgerät für die Bestimmung von Glucose in einer Vollblutprobe unter Verwendung eines analytischen Teststreifens auf elektrochemischer Basis gestaltet ist.

3. Verfahren zum Verwenden eines handgeführten Prüfmessgeräts, das für die Bestimmung eines Analyten in einer Körperfluidprobe gestaltet ist, wobei das Verfahren umfasst:
Verwenden eines elektromagnetische-Störung-Erkennungsschaltungsblocks, der eine Antenne, die zum Erfassen elektromagnetischer Felder mit einer vorbestimmten Frequenz gestaltet ist, des handgeführten Prüfmessgeräts umfasst, um ein Signal zu erzeugen, das für ein von der Antenne erfasstes elektromagnetisches Feld repräsentativ ist, und das Signal an einen Prüfmessgerät-Steuerschaltungsblock des handgeführten Prüfmessgeräts zu übermitteln; und
Unterbrechen des Betriebs des handgeführten Prüfmessgeräts, wenn das von dem elektromagnetische-Störung-Erkennungsschaltungsblock empfangene Signal für ein vorbestimmtes elektromagnetisches Feld repräsentativ ist, das den Betrieb des handgeführten Prüfmessgeräts stört;
wobei das vorbestimmte elektromagnetische Feld ein elektromagnetisches Feld ist, das eine Feldstärke von höher als 10 V/m aufweist, und entweder:
eine Frequenz in dem Bereich von 800 MHz bis 2200 MHz aufweist; oder
ein amplitudenmoduliertes elektromagnetisches Feld mit Radiofrequenz (RF) ist, und das von dem elektromagnetische-Störung-Erkennungsschaltungsblock empfangene Signal ein demoduliertes Signal ist; oder
ein pulsmoduliertes elektromagnetisches RF-Feld ist, und das von dem elektromagnetische-Störung-Erkennungsschaltungsblock empfangene Signal ein demoduliertes Signal ist.

4. Verfahren gemäß Anspruch 3, ferner umfassend:
Aufbringen einer Körperfluidprobe auf einen analytischen Teststreifen auf elektrochemischer Basis;
Messen einer elektrochemischen Antwort des analytischen Teststreifens auf elektrochemischer Basis unter Verwendung des handgeführten Prüfmessgeräts; und
Bestimmen des Analyten auf der Grundlage der gemessenen elektrochemischen Antwort.

5. Verfahren gemäß Anspruch 4, wobei die Körperfluidprobe eine Vollblutprobe ist und der Analyt Glucose ist.

6. Handgeführtes Prüfmessgerät gemäß Anspruch 1 oder Verfahren gemäß Anspruch 3, wobei der Prüfmessgerät-Steuerschaltungsblock ein Mikrocontrollerblock ist.

7. Verfahren gemäß Anspruch 3, wobei der Schritt des Unterbrechens das Unterbrechen des Betriebs des handgeführten Prüfmessgeräts durch Anzeigen einer elektromagnetische-Störung-Warnmeldung auf einer Anzeige des handgeführten Prüfmessgeräts umfasst.

8. Handgeführtes Prüfmessgerät gemäß Anspruch 1 oder Verfahren gemäß Anspruch 3, wobei der elektromagnetische-Störung-Erkennungsschaltungsblock ferner als AM-Demodulator gestaltet ist, der ein 217-Hz-Signal erzeugt, das für ein von einem GSM-Mobiltelefon erzeugtes und von der Antenne erfasstes elektromagnetisches Feld repräsentativ ist.

9. Handgeführtes Prüfmessgerät gemäß Anspruch 6, ferner umfassend eine Anzeige und einen Anzeigenmodulblock, und
wobei der Mikrocontrollerblock, der Anzeigemodulblock und die Anzeige dafür gestaltet sind, einem Anwender eine Warnmeldung anzuzeigen, wenn das von dem elektromagnetische-Störung-Erkennungsschaltungsblock empfangene Signal für ein vorbestimmtes elektromagnetisches Feld repräsentativ ist, das den Betrieb des handgeführten Prüfmessgeräts stört.

## Revendications

1. Dispositif de mesure de test portatif (100) pour utilisation avec une bandelette de test analytique dans la détermination d'un analyte dans un échantillon de fluide corporel, le dispositif de mesure de test portatif comprenant :
un boîtier (110) ;
un bloc de circuit de commande de dispositif de mesure de test (114) ; et
un bloc de circuit de détection d'interférence électromagnétique (112) qui comprend :
une antenne (122) configurée pour détecter des champs électromagnétiques d'une fréquence prédéterminée ;
dans lequel le bloc de circuit de détection d'interférence électromagnétique est configuré pour générer un signal représentatif d'un champ électromagnétique détecté par l'antenne et pour fournir le signal au bloc de circuit de commande de dispositif de mesure de test ; et
dans lequel le bloc de circuit de commande de dispositif de mesure de test est configuré pour interrompre le fonctionnement du dispositif de mesure de test portatif quand le signal reçu provenant du bloc de circuit de détection d'interférence électromagnétique est représentatif d'un champ électromagnétique prédéterminé qui interfère avec le fonctionnement du dispositif de mesure de test portatif ;
dans lequel ledit champ magnétique prédéterminé est un champ électromagnétique qui a une force de champ supérieure à 10 V/m et soit :
a une fréquence située dans la plage allant de 800 MHz à 2200 MHz ; soit
est un champ électromagnétique de radiofréquence (RF) modulé en amplitude et le signal reçu provenant du bloc de circuit de détection d'interférence électromagnétique est un signal démodulé ; soit
est un champ électromagnétique RF modulé en impulsion et le signal reçu provenant du bloc de circuit de détection d'interférence électromagnétique est un signal démodulé.

2. Dispositif de mesure de test portatif selon la revendication 1, lequel dispositif de mesure de test portatif est configuré pour la détermination du glucose dans un échantillon de sang entier en utilisant une bandelette de test analytique basée sur un processus électrochimique.

3. Procédé pour employer un dispositif de mesure de test portatif configuré pour la détermination d'un analyte dans un échantillon de fluide corporel, le procédé comprenant :
l'emploi d'un bloc de circuit de détection d'interférence électromagnétique, qui comprend une antenne configurée pour détecter des champs électromagnétiques d'une fréquence prédéterminée, du dispositif de mesure de test portatif pour générer un signal représentatif d'un champ électromagnétique détecté par l'antenne et pour fournir le signal à un bloc de circuit de commande de dispositif de mesure de test du dispositif de mesure de test portatif ; et
l'interruption du fonctionnement du dispositif de mesure de test portatif quand le signal reçu par le bloc de commande de circuit de dispositif de mesure de test, provenant du bloc de circuit de détection d'interférence électromagnétique, est représentatif d'un champ électromagnétique prédéterminé qui interfère avec le fonctionnement du dispositif de mesure de test portatif,
dans lequel ledit champ magnétique prédéterminé est un champ électromagnétique qui a une force de champ supérieure à 10 V/m et soit :
a une fréquence située dans la plage allant de 800 MHz à 2200 MHz ; soit
est un champ électromagnétique de radiofréquence (RF) modulé en amplitude et le signal reçu provenant du bloc de circuit de détection d'interférence électromagnétique est un signal démodulé ; soit
est un champ électromagnétique RF modulé en impulsion et le signal reçu provenant du bloc de circuit de détection d'interférence électromagnétique est un signal démodulé.

4. Procédé selon la revendication 3, comprenant en outre :
l'application d'un échantillon de fluide corporel à une bandelette de test analytique basée sur un processus électrochimique ;
la mesure de la réponse électrochimique de la bandelette de test analytique basée sur un processus électrochimique par utilisation du dispositif de mesure de test portatif ; et
la détermination de l'analyte sur la base de la réponse électrochimique mesurée.

5. Procédé selon la revendication 4, dans lequel l'échantillon de fluide corporel est un échantillon de sang entier et l'analyte est le glucose.

6. Dispositif de mesure de test portatif selon la revendication 1 ou procédé selon la revendication 3, dans lequel le bloc de circuit de commande de dispositif de mesure de test est un bloc de microcontrôleur.

7. Procédé selon la revendication 3, dans lequel l'étape d'interruption comprend l'interruption du fonctionnement du dispositif de mesure de test portatif par affichage d'un message d'alerte d'interférence électromagnétique sur un système d'affichage du dispositif de mesure de test portatif.

8. Dispositif de mesure de test portatif selon la revendication 1 ou procédé selon la revendication 3, dans lequel le bloc de circuit de détection d'interférence électromagnétique est en outre configuré comme un démodulateur AM qui produit un signal de 217 Hz représentatif d'un champ électromagnétique créé par un téléphone portable GSM et détecté par l'antenne.

9. Dispositif de mesure de test portatif selon la revendication 6, comprenant en outre un système d'affichage et un bloc de module d'affichage, et
dans lequel le bloc de microcontrôleur, le bloc de module d'affichage et le système d'affichage sont configurés pour afficher un message d'alerte à un utilisateur quand le signal reçu provenant du bloc de circuit de détection d'interférence électromagnétique est représentatif d'un champ électromagnétique prédéterminé qui interfère avec le fonctionnement du dispositif de mesure de test portatif.
